# EUROPEAN PATENT SPECIFICATION

(11) **EP 4 474 001 B1**
(45) Date of publication and mention of the grant of the patent: **11.02.2026**
(21) Application number: 23178090.9
(22) Date of filing: 07.06.2023
(51) Int. Cl.: A61N 1/05, A61B 5/24

(54) **APPARATUS AND SYSTEM FOR STIMULATING AND / OR RECORDING SIGNALS TRAVELLING ALONG NERVE FIBERS**
VORRICHTUNG UND SYSTEM ZUR STIMULATION UND/ODER AUFZEICHNUNG VON SIGNALEN, DIE SICH ENTLANG NERVENFASERN BEWEGEN
APPAREIL ET SYSTÈME DE STIMULATION ET/OU D'ENREGISTREMENT DE SIGNAUX SE DÉPLAÇANT LE LONG DE FIBRES NERVEUSES

(43) Date of publication of application: 11.12.2024
(73) Proprietor: CereGate GmbH, 81671 München (DE)
(72) Inventor: VÁRKUTI, Bálint, 81679 München (DE); HAGH GOOIE, Saman, 81671 München (DE); MANOLA, Ljubomir, 81671 München (DE)
(74) Representative: Bardehle Pagenberg Partnerschaft mbB Patentanwälte Rechtsanwälte

(56) References cited:
- EP-A1- 4 070 851
- US-A1- 2016 206 873
- US-A1- 2016 339 251
- US-A1- 2019 366 077
- US-A1- 2021 187 284
- US-B1- 9 216 282

## Description

### 1. Technical field

The present disclosure relates to an apparatus, system, device and a non-transitory computer readable memory medium for stimulating and / or recording of signals travelling along nerve fibers of a spinal cord. In particular, the present disclosure relates to an improved electrode arrangement comprised by such an apparatus, system, and device.

### 2. Technical background

Computer to spinal cord interface systems (CSI) can be applied in a variety of fields in order to assist humans and to improve their wellbeing. For instance, CSI are used to communicate information to a human, to assist a human in performing certain tasks, e.g. by enrichment of the environment with virtual or additive reality, to provide therapy in medical or psychological conditions and to provide rehabilitation for faster disease recovery. Further applications are treatment of chronic pain conditions. It is expected that the number of humans that could benefit from CSI patients increases, such that a demand for CSI is also likely to increase.

CSI provide stimulation to the central nervous system (CNS) by means of electrical fields, which are provided by one or more electrodes. The electrodes are in conductive connection with a neurostimulation device via leads. Further, the electrodes are usually provided at the end of the leads or on a substrate at the end of the leads. The neurostimulation device provides sequences of neurostimulation pulses to the stimulation leads and, thereby, to the electrodes.

Presently available stimulation leads can be categorized into two common types, namely percutaneous leads and paddle leads. The implantation procedure of percutaneous leads is relatively simple and is performed as an out-patient process. Percutaneous leads often include electrodes that correspond in shape to the shape of the lead, wherein the lead usually has a small profile. To the contrary, paddle leads require a laminectomy procedure for implantation and is, thus, more invasive. Paddle leads offer the possibility of arranging a plurality of electrodes on the body of the lead. Thereby, improved stimulation can be achieved. Furthermore, due to the shape of the paddle lead an improved positioning can also be ensured.

However, it is also desirable that electrodes / leads are able to record nerve fibers of the spinal cord of a human. Recording of an evoked compound action potential (ECAP) has the benefit to be used for closed loop applications, which can improve stimulation of nerve fibers. It is also desirable to target specific sensory tracts of the spinal cord. There is also a demand to control the depth of stimulation by way of the electrodes / leads. Moreover, it is desirable to reduce movement of paddle leads (lead migration). Such movement often occurs due to patient's movement and / or change of position.

Conventional implementations of laminectomy leads have arrangements of multiple columns and multiple rows in an attempt to address one or more of the above needs and demands. The following documents may exemplarily be mentioned as background art.

US 7,979,131 B2 relates to a paddle lead that is implanted within a patient such that the electrodes are positioned within the cervical or thoracic spinal levels. A first row of electrodes is arranged to be effective for a first pain location. A second row of electrodes is arranged to be effective for a second pain location with minimal effects on other regions of the body. An IPG is programmed to deliver pulses using the rows of electrodes.

US 2018 / 0200505 A1 relates to a method assisting a person by providing an electrode array having a substrate supporting a plurality of stimulation contacts configured to stimulate at least a portion of the spinal cord neural tissue. According to this document, conventional electrodes often are thick and bulky, which limits their ability to be positioned relatively significant distances from the anatomical midline. Thus, a flexible electrode array is said to be provided having a substrate supporting a plurality of stimulation contacts configured to stimulate at least a portion of the spinal cord neural tissue and / or dorsal root(s).

US 2019/0001122 A1 relates to a lead for neuromodulation and / or neurorecording. The lead comprises at least one lead cable, at least one electrode section and at least one electrode placed in the electrode section. The lead further comprises an anchor, which is pre-attached to the lead and configured to engage with tissue fixation apparatus and / or hold a tissue fixation. This documents claims that the provided lead can be fixed at the implantation site with high accuracy and that the fixation is easy to establish.

US 2019/0366077 A1 relates to an electrode array for neuromodulation, comprising a first electrode section with more than two electrodes being arranged parallel and densely packed in the first electrode section. The array further comprises a second electrode section with more electrodes than in the first electrode section. The electrodes in the second electrode section are arranged symmetrically with respect to the longitudinal axis and transversal offset to each other. Document US D907,206 S shows further figures with regard to the aforementioned documents. US 2021/187284 A1, US 9 216 282 B1 and US 2016/206873 A1 discuss further electrode arrays.

### 3. Summary

In view of the foregoing, a new apparatus for stimulating and / or recording signals traveling along nerve fibers of a spinal cord and respective systems, devices and mediums are desired. The stimulation and recording of signals traveling along nerve fibers should thereby be improved. This and similar problems are at least partially solved by the subject-matter of the appended claims.

Specifically, aspects of the present disclosure relate to an apparatus for stimulating and / or recording signals travelling along nerve fibers of a spinal cord, comprising one or more first electrode arrangements arranged on an elongated and non-conducting base material, each first electrode arrangement comprising: an elongated first central reference electrode extending along a longitudinal direction of the elongated base material; and a plurality of first electrodes arranged on both sides of the elongated first central reference electrode; wherein a length of the elongated first central reference electrode is at least two times larger or at least three times larger than an average length of the plurality of first electrodes; and wherein the elongated first central reference electrode is configured to provide a counter potential for stimulating and / or a reference potential for recording of the nerve fibers of the spinal cord via the plurality of first electrodes.

Further aspects of the present disclosure relate to apparatus for stimulating and / or recording signals travelling along nerve fibers of a spinal cord, comprising one or more second electrode arrangements arranged on an elongated and non-conducting base material, each second electrode arrangement comprising: an elongated second central reference electrode extending along a width direction of the elongated base material, substantially perpendicular to a longitudinal direction of the elongated base material; and a plurality of second electrodes arranged in one or more rows on one or on both sides of the second elongated central reference electrode; wherein a distance between the second electrodes and the elongated second central reference electrode is substantially the same for all second electrodes of the same row.

Further aspects of the present disclosure relate to an apparatus for stimulating and / or recording signals travelling along nerve fibers of a spinal cord, comprising: two or more electrode arrangements arranged on an elongated and non-conducting base material; wherein each electrode arrangement comprises a plurality of electrodes; and wherein a distance between a first electrode arrangement and a neighboring second electrode arrangement of the two or more electrode arrangements is larger than a distance of all neighboring pairs of electrodes of the same electrode arrangement.

Further aspects of the present disclosure relate to an apparatus for stimulating and / or recording signals traveling along nerve fibers of a spinal cord, comprising: one or more electrode arrangements arranged on an elongated and non-conducting base material; wherein the elongated base material comprises a guiding tunnel configured to receive and guide at least one wire element, preferably a wire electrode, more preferably to at least partially surround the spinal cord.

Further aspects of the present disclosure relate to a system for stimulating and / or recording signals traveling along nerve fibers of a spinal cord, comprising the apparatus as described in here and particularly as described in the foregoing aspect; and at least one partially insulated wire electrode comprising one or more electric contacts configured such that at least some of the electric contacts reside at the left side and / or on the right side and / or on the ventral side of the spinal cord, when the wire electrode is inserted into the guiding tunnel. This insertion may be understood as advancing the wire electrode through the guiding tunnel.

Further aspects of the present disclosure relate to an apparatus for stimulating and / or recording signals traveling along nerve fibers of a spinal cord, comprising: one or more electrode arrangements arranged on an elongated and non-conducting base material; and one or more protrusions, preferably wings, extending from the base material in a width direction, wherein the one or more protrusions are configured to at least partially surround the spinal cord and comprise one or more electric contacts configured to reside at the left side and / or on the right side and / or on the ventral side of the spinal cord; and / or one or more protrusions, extending from the base material such that a force is provided pushing the apparatus towards the spinal cord during use.

Further aspects of the present disclosure relate to an apparatus for stimulating and / or recording signals travelling along nerve fibers of a spinal cord, comprising: one or more electrode arrangements arranged on an elongated and non-conducting base material, each electrode arrangement comprising: a plurality of electrodes; a reference electrode substantially surrounding the plurality of electrodes; wherein the reference electrode is configured to provide a reference counter potential for stimulating and / or a reference potential for recording of the nerve fibers of the spinal cord via the plurality of electrodes.

Further aspects of the present disclosure relate to a neurostimulation device for stimulating and / or recording signals travelling along nerve fibers of a spinal cord, the device comprising: the apparatus of any of the aspects described herein; and neurostimulation and recoding circuitry operably connected to the electrodes of the apparatus.

Further aspects of the present disclosure relate to a computer program, comprising instructions for causing a neurostimulation device to carry out the following steps when being executed by signal processing circuitry of the neurostimulation device: outputting a first neurostimulation signal to a first electrode arrangement of a spinal cord stimulation and recording apparatus, wherein the neurostimulation signal is configured to elicit action potentials in afferent sensory nerve fibers of a spinal cord; recording, from a second electrode arrangement of the spinal cord stimulation and recording apparatus, an evoked compound action potential, ECAP, propagating antidromically and/or orthodromically along the stimulated afferent nerve fibers; analyzing the recorded antidromic ECAP; and outputting, based on the analyzed and recorded antidromic ECAP, a second neurostimulation signal to the first electrode arrangement, wherein the second neurostimulation signal is configured to elicit action potentials in the afferent sensory nerve fibers of the spinal cord.

### 4. Brief description of the figures

Various aspects of the present disclosure are described in more detail in the following by reference to the accompanying figures. These figures show:
- **Fig. 1**: a schematic illustration of a general setup of an apparatus for stimulating and / or recording signals traveling along spinal nerve fibers when provided in a patient according to aspects of the present disclosure;
- **Fig. 2**: a schematic illustration of an apparatus for stimulating and / or recording signals traveling along spinal nerve fibers according to aspects of the present disclosure;
- **Fig. 3**: a schematic illustration of an apparatus for stimulating and / or recording signals traveling along spinal nerve fibers according to further aspects of the present disclosure;
- **Fig. 4**: a schematic illustration of an apparatus for stimulating and / or recording signals traveling along spinal nerve fibers according to yet further aspects of the present disclosure;
- **Fig. 5**: a schematic illustration of an apparatus for stimulating and / or recording signals traveling along spinal nerve fibers according to yet further aspects of the present disclosure;
- **Fig. 6**: a schematic illustration of an apparatus for stimulating and / or recording signals traveling along spinal nerve fibers according to yet further aspects of the present disclosure;
- **Fig. 7**: a schematic illustration of an apparatus for stimulating and / or recording signals traveling along spinal nerve fibers according to yet further aspects of the present disclosure in three different perspectives;
- **Fig. 8**: a schematic illustration of an apparatus for stimulating and / or recording signals traveling along spinal nerve fibers according to yet further aspects of the present disclosure;
- **Fig. 9**: a schematic illustration of an apparatus for stimulating and / or recording signals traveling along spinal nerve fibers according to yet further aspects of the present disclosure;
- **Fig. 10**: a schematic illustration of an apparatus for stimulating and / or recording signals traveling along spinal nerve fibers according to yet further aspects of the present disclosure;
- **Fig. 11**: a functional block circuit diagram illustrating an exemplary neurostimulation device according to an aspect of the present disclosure.

### 5. Detailed description of the figures

While specific feature combinations are described in the following with respect to exemplary aspects of the present disclosure, it is to be understood that not all features of the described aspects must be present for realizing the technical advantages provided by the devices, systems, methods and computer-readable media provided by the present disclosure. The disclosed aspects may be modified by combining certain features of one aspect with one or more features of another aspect if technically feasible and functionally compatible. Specifically, the skilled person will understand that features, steps, components and / or functional elements of one aspect can be combined with technically compatible features, steps, components and / or functional elements of any other aspect of the present disclosure.

Moreover, the described modules, functions, functional elements of the devices and systems disclosed herein can be implemented in hardware, in software, or a combination thereof. For instance, the described modules, functions, functional elements of the devices and systems disclosed herein may be implemented via application specific hardware components such as application specific integrated circuits, ASICs, and / or field programmable gate arrays, FPGAs, and / or similar components and / or application specific software modules, applications, or subroutines being executed on multi-purpose data and signal processing circuitry such as CPUs, DSPs and / or systems on a chip (SOCs) or similar components or any combination thereof. For instance, the various modules, functional elements, etc. described herein may be implemented on a multi-purpose data and signal processing device configured for executing application specific software modules and for communicating with various sensor devices and / or neurostimulation devices or systems via conventional wireless communication interfaces such as a Near Field Communication (NFC), a WIFI and / or a Bluetooth interface. For example, aspects of the programmer devices described herein may be implemented on a smart phone or a tablet computer or a similar computing device. Alternatively, the various modules, functional elements, etc. described herein may also be part of an integrated neurostimulation device, apparatus or system, comprising specialized electronic circuitry (e.g., neurostimulation signal generators, amplifiers etc.) for generating and applying stimulation and / or recording signals travelling along nerve fibers of a spinal cord of the individual (e.g., a multi-contact electrode, a spinal cord stimulation electrode).

The term "based on" as used herein, shall not be construed as a reference to a closed set of information, one or more conditions, one or more factors, or the like. In other words, the phrase "based on A" (where "A" may be information, a condition, a factor, or the like) shall be construed as "based at least on A" unless specifically recited differently. When reference is made herein to a "component", "unit", "device" or the like, this should not be understood as limiting to a particular "component", "unit", "device" or the like, but should encompass equivalents that could have similar and / or the same functions.

### General setup

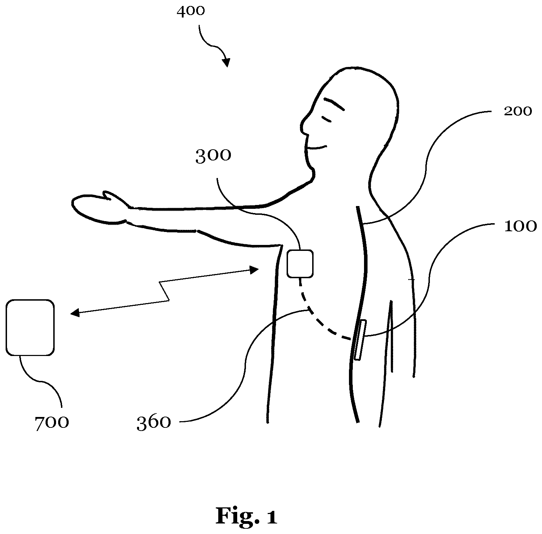

**Fig. 1** shows a schematic illustration of an apparatus 100 for stimulating and / or recording signals travelling along nerve fibers of a spinal cord 200 according to aspects of the present disclosure. A person 400 is depicted, which has been implanted with said apparatus 100. The apparatus 100 is described elsewhere in greater detail and comprises electrodes.

The person 400 is further equipped with a neurostimulation device 300, that may be an implantable and programmable pulse generator (IPG), which may be implanted under the skin of the person 400. Alternatively, the neurostimulation device 300 may be arranged somewhere else or in the vicinity of the body of the person 400. In one example, the neurostimulation device 300 may be in wireless communication (e.g., via a Bluetooth, WI-FI, NFC or a similar wireless interface technology) with a control device, that may be implemented by a dedicated signal and data processing device such as a smartphone or a similar electronic information processing device. The neurostimulation device 300 may also comprise a rechargeable power source, which may for instance be wirelessly charged via a wireless charging interface.

Further, a programmer device 700 is depicted for configuring the neurostimulation device 300 which may be comprised by the control device or a separate device such as a personal computer, tablet computer or the like. The programmer device 700 may exchange data and control instructions with the neurostimulation device 300, for example via a wireless communication interface. Programming of the neurostimulation device 300 may be for instance performed through a physicians' interaction but is not limited thereto.

Further, leads 360 from neurostimulation device 300 are directed towards an apparatus 100 for stimulating and / or recording signals traveling along nerve fibers as described elsewhere in greater detail. The apparatus 100 is in proximity of the spinal cord 200.

### Reference electrode along longitudinal direction

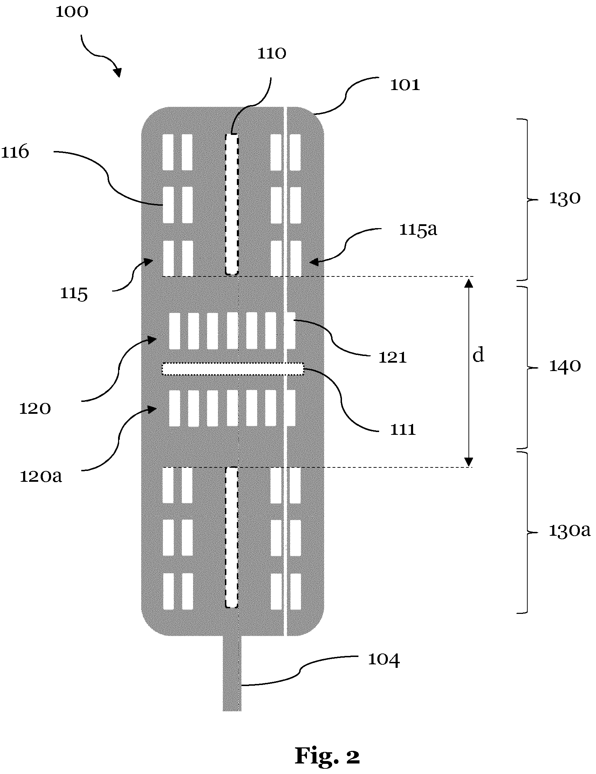

**Fig. 2** shows a schematic illustration of an apparatus 100 for stimulating and / or recording signals traveling along nerve fibers according to aspects of the present disclosure. The apparatus 100 comprises one or more first electrode arrangements arranged on an elongated and non-conducting base material 101. Each first electrode arrangement has an elongated first central reference electrode 110 extending along a longitudinal direction of the elongated base material 101. Further, each first electrode arrangement has a plurality of first electrodes 115, 115a arranged on both sides of the elongated first central reference electrode 110. The length of the elongated first central reference electrode 110 is larger, for instance at least two times larger or at least three times larger than an average length of the plurality of first electrodes 115, 115a. Moreover, the elongated first central reference electrode 110 is configured to provide a counter potential for stimulating and / or a reference potential for recording of the nerve fibers of the spinal cord via the plurality of first electrodes.

The apparatus 100 can provide for stimulation causing signals traveling along nerve fibers, in particular signals travelling along afferent nerve fibers. In the peripheral nervous system, afferent and efferent nerve fibers pertain and are part of the somatic nervous system. Both fibers arise from outside of the spinal cord 200. The afferent fibers are carried into the spinal cord 200 by sensory nerves. Further, efferent fibers are carried out of the spinal cord 200 by motor nerves in the direction to muscles. When afferent sensory nerve fibers are stimulated a sensory percept in the cortex of the person 400 is elicited that can indicate any kind of somatosensory information to the person 400.

This arrangement facilitates an improved placement of the electrodes with respect to the spinal cord 200 of the person 400. This allows to stimulate specific dermatome zones more accurately. Such an improved stimulation of specific dermatome zones/fibers/levels enables that specific perceptual channels can be elicited better and / or more consistently. It has been shown in the prior art that large stimulation artifacts in the recorded signals are caused by inadequate and / or suboptimal placement of the electrodes. Such inadequate and / or suboptimal placement is even exacerbated in conventional designs due to movements of a person. Such movements of the apparatus may amount for instance to about 2.2 mm or more or less. This disadvantage can be overcome by the aspects described in accordance with the present disclosure. In an example, the apparatus may be used for pain suppression. In another example, it could be used to target specific sensory tracts in the spinal cord 200 for transmission of information. Said information may be transmitted into afferent axons of the spinal cord 200 of the person 400.

In addition, the central reference electrode allows to improve recording of evoked compound action potential (ECAP). For instance, antidromic and / or orthodromic ECAP can be recorded and may then be used for a closed loop control. That is, because stimulation may lead to elicitation of action potentials that are propagating orthodromically and antidromically. It may be possible to use the recorded ECAP to perform a recalibration in order to improve elicitation of desired perceptual channels.

The first reference electrode 110 is a central reference electrode 110, for providing a counter potential for stimulating, which allows for more control over the lateral stimulation and simultaneous recording from the contralateral side which would give a more localized stimulation and/or recording.

Moreover, the apparatus 100 provides for more control of the spinal cord stimulation. For instance, more control may be provided over the depth and medio-lateral distribution. This provides for more localized lateral or dermatome level stimulation and recording.

The apparatus 100 may be provided with a lead 104 for providing electrical current to the electrodes. The elongated and non-conducting base material 101 could be any suitable base material including but not limited to silicon, thin-film Teflon. Further, the material 101 could be any bio-compatible material.

As can be further seen in Fig. 1, each electrode (exemplarily, merely one electrode 116 is indicated) of the plurality of first electrodes 115, 115a comprise an elongated shape. The longitudinal direction of each electrode 116 of the plurality of first electrodes 115, 115a is substantially parallel to the longitudinal direction of the first central reference electrode 110.

Further, the plurality of first electrodes 115, 115a is arranged in a line-symmetric manner with respect to a central line of the elongated first central reference electrode 110. Each first electrode arrangement 130, 130a comprises three or more pairs of first electrodes 116 on both sides of the first central reference electrode 110. In particular, when reviewing Fig. 2, there are three pairs of first electrodes 116 on the left and on the right-hand side of the first central reference electrode 110.

Each electrode 116 of the plurality of first electrodes 115, 115a can be controlled independently to stimulate and / or record from the nerve fibers of the spinal cord. This enhances stimulation of dermatome fibers and elicitation of perceptual channels and / or recording of the amplitudes of ECAPs, as dedicated mediolateral and rostrocaudal locations of stimulation can be controlled. In particular, this independent control enables flexibility in terms of choosing a reference / ground configuration during operation of the apparatus 100. This may be of importance in various use cases including but not limited to stimulation trials required for mapping the location and intensity of corresponding somatosensory dermatomes. However, it is emphasized that this merely serves as an example to contribute to an understanding of one of various use cases of the apparatus 100.

In various examples and as shown in Fig. 2, the apparatus 100 comprises at least two first electrode arrangements 130, 130a, wherein a distance (indicated as "d" in Fig. 2) between the two first electrode arrangements 130, 130a in the longitudinal direction of the elongated base material 101 corresponds to a typical distance in a longitudinal direction of the spinal cord between a center of a first dorsal root entry zone and a second dorsal root entry zone.

The skilled person is aware of such a typical distance between a center of a first dorsal root entry zone and a second dorsal root entry zone. Selecting such a distance d for the least two first electrode arrangements 130, 130a has proven to be advantageous in that specific dermatome zones can be stimulated. In particular, as distances between dorsal roots may differ along the spinal cord and / or between humans, it is beneficial to adjust the distance "d" (indicated in Fig. 2 as the distance from one edge of the other edge of the electrode arrangements 130, 130a) accordingly as described in here. Such distances d may be at least 0.5 cm, 1 cm, 2 cm, 3 cm, 4 cm or 5 cm and / or at most 6 cm, 5 cm, 4 cm, 3 cm, 2 cm or 1 cm. Further, the typical distances between dorsal roots along the spinal cord in humans may differ depending on the region of the spinal cord. For instance, in the cervical (neck) region, the distance may be approximately 8 mm, while in the thoracic (chest) region, the distance may be approximately 11 mm. In the lumbar (lower back) region, the distance may be approximately 17 mm, and in the sacral (pelvic) region, the distance may be approximately 21 mm. It is understood that these numbers merely serve as examples that that the distances described herein are not limited to these.

As can be further seen in Fig. 2, the apparatus comprises one or more second electrode arrangements 140 (only one 140 is shown in Fig. 2) arranged on the elongated base material 101. Each second electrode arrangement 140 is comprising: an elongated second central reference electrode 111 extending along a width direction of the elongated base material 101, substantially perpendicular to the longitudinal direction of the elongated base material 101. It is understood that the longitudinal direction of the elongated base material 101 may be in the direction along the spinal cord. Each second electrode arrangement 140 is further comprising a plurality of second electrodes 120, 120a arranged in one or more rows on one or on both sides of the elongated second central reference electrode 111. As can be seen, a distance between the second electrodes 120, 120a and the elongated second central reference electrode 111 is substantially the same for all second electrodes of the same row (one of the plurality of second electrodes is indicated as 121).

In one example, one or more of the first electrode arrangements 130, 130a is configured for stimulating afferent sensory nerve fibers of the spinal cord. Additionally or alternatively one or more of the second electrode arrangements 140 is configured for recording ECAPs, elicited via the first electrode arrangement(s) 130, 130a.

In another example, one or more of the second electrode arrangements 140 is configured for stimulating afferent sensory nerve fibers of the spinal cord. Additionally or alternatively one or more of the first electrode arrangements 130, 130a is configured for recording ECAPs, elicited via the second electrode arrangement(s) 140. In this manner the flexibility of the apparatus 100 is increased.

### Reference electrode along width direction

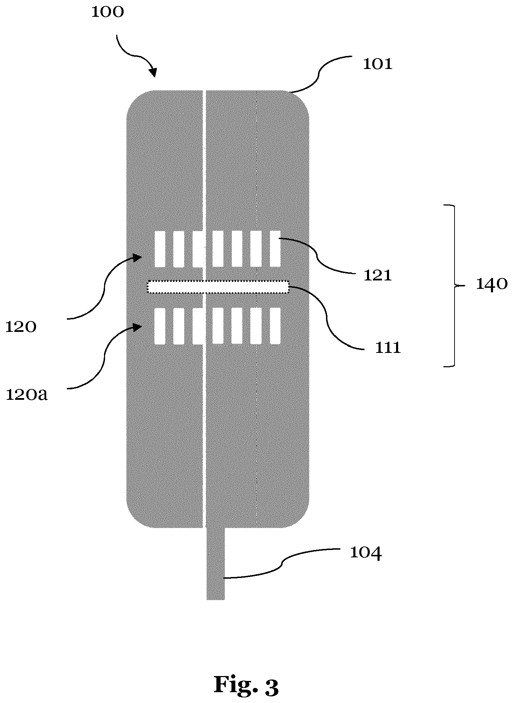

**Fig. 3** shows a schematic illustration of an apparatus 100 for stimulating and / or recording signals traveling along nerve fibers according to further aspects of the present disclosure. The apparatus 100 comprises one or more second electrode arrangements 140 arranged on an elongated and non-conducting base material 101, each second electrode arrangement 140 comprises the following: an elongated second central reference electrode 111 extending along a width direction of the elongated base material 101, substantially perpendicular to a longitudinal direction of the elongated base material 101. Each second electrode arrangement 140 also comprises a plurality of second electrodes 120, 120a arranged in one or more rows on one or on both sides of the second elongated central reference electrode 111. A distance between the second electrodes (one of which is indicated as 121) and the elongated second central reference electrode 111 is substantially the same for all second electrodes 121 of the same row.

Further, Fig. 3 shows that each electrode 121 of the plurality of second electrodes 120, 120a comprises an elongated shape and the longitudinal direction of each electrode 121 of the plurality of second electrodes 120, 120a (which is directed along the length direction of the base material 101) is substantially orthogonal to the longitudinal direction of the second central reference electrode 111 (which is directed along the width direction of the base material 101).

The plurality of second electrodes 120, 120a may be arranged in a line-symmetric manner with respect to a central line of the elongated second central reference electrode 111. Further, each row of the second electrode arrangement 140 comprises at least three, or at least five second electrodes 121 being arranged across a central line of the spinal cord. In this particular example of Fig. 3, each row comprises seven second electrodes 121.

Moreover, each second electrode 121 can be controlled independently for stimulating and / or recording of the nerve fibers of the spinal cord. Respective advantages as set forth above with respect to the first electrode arrangements 130, 130a apply in here as well.

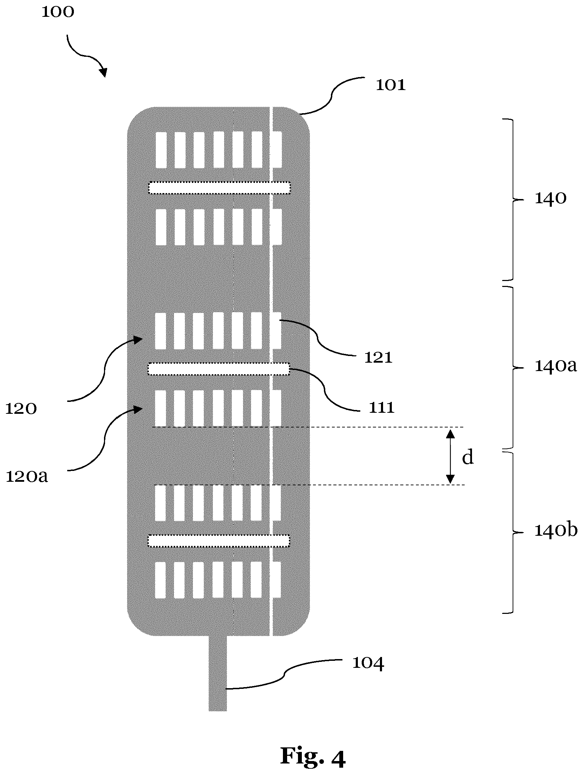

**Fig. 4** shows a schematic illustration of an apparatus 100 for stimulating and / or recording signals traveling along nerve fibers according to yet further aspects of the present disclosure. This apparatus 100 is similar to the one shown in Fig. 3. In here, the apparatus 100 comprises at least two second electrode arrangements 140, 140a (in particular three second electrode arrangements 140, 140a, 140b are shown). Further, a distance between two second electrode arrangements, i.e., between the (first) second electrode arrangement 140 and the (second) second electrode arrangement 140a and / or the distance between the (second) second electrode arrangement 140a and the (third) second electrode arrangement 140b in the longitudinal direction of the elongated base material 101 corresponds to a typical distance in a longitudinal direction of the spinal cord between a center of a first dorsal root entry zone and a second dorsal root entry zone. This distance is indicated in Fig. 4 as "d".

This facilitates stimulation and / or recording from a medial / deep part of the spinal cord. In particular, as distances between dorsal roots may differ, it is beneficial to adjust the distance of the electrode arrangements 140a, 140b accordingly.

It is possible to combine the second electrode arrangements 140, described with reference to Figs. 3 and 4 with the one or more first electrode arrangements 130, 130a described with reference to Fig. 2. In this manner, the apparatus 100 of Fig. 4 is further comprising one or more first electrode arrangements 130, 130a arranged on the elongated base material 101, each first electrode arrangement 130, 130a is comprising: an elongated first central reference electrode 110 extending along a longitudinal direction of the elongated base material 101. Further, the apparatus 100 comprises a plurality of first electrodes 115, 115a arranged on both sides of the elongated first central reference electrode 110. Moreover, a length of the elongated first central reference electrode 110 is at least two times larger or at least three times larger than an average length of the plurality of first electrodes 115 115a. Further, the elongated first central reference electrode 110 is configured to provide a reference potential for stimulating and / or for recording of the nerve fibers of the spinal cord via the plurality of first electrodes 115, 115a.

### Multiplexer

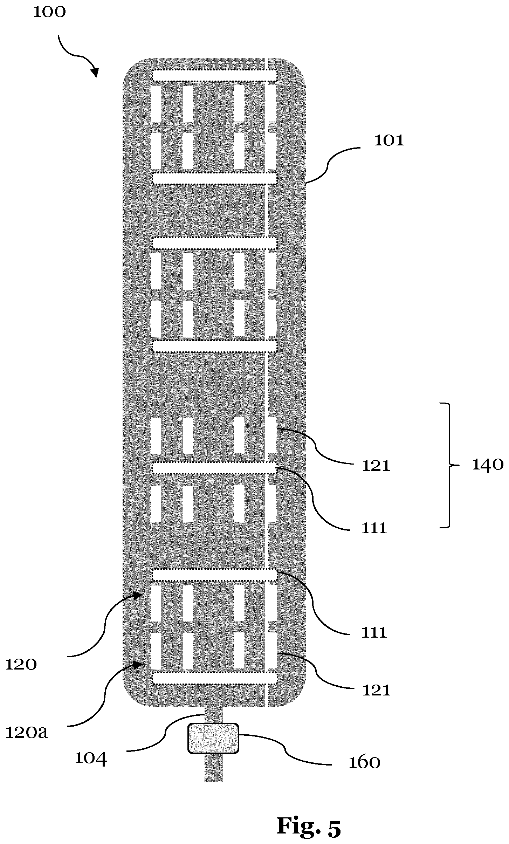

**Fig. 5** shows a schematic illustration of an apparatus 100 for stimulating and / or recording signals traveling along nerve fibers according to yet further aspects of the present disclosure. This apparatus 100 may be any apparatus 100 as described in here. Further the apparatus 100 comprises a remote controllable multiplexer 160, operably connected to the first and / or second electrodes 116, 121 and configured to multiplex stimulation and / or recording via the first 116 and / or second 121 electrodes. The multiplexer 160 may be connected to all electrodes of the apparatus 100. The multiplexer 160 may in one example be implemented by a chip added to one or more first 116 and / or second 121 electrodes. The multiplexer 160 may be configured to select a subset of one or more first 116 and / or second 121 electrodes in order to control these for stimulating and / or recording. Further, the multiplexer 160 may be controlled by employing a neurostimulation device 300 (as best seen in Fig. 1), such as an IPG.

Exemplarily, a second electrode arrangement 140 is indicated in Fig. 5, which comprises one second central reference electrode 111 for dermatome specific ECAP detection. This second central reference electrode 111 can be used for the eight neighboring electrodes 121 (only one of which is indicated for illustration purposes), four of which 121 are disposed on the upper side of the reference electrode 111 and four of which 121 are disposed on the lower side of the reference electrode 111.

### Form factors

Various embodiments of the apparatus 100 are encompassed in the present disclosure having a design that allows for easier implantation and enhanced bandwidth as detailed in the following.

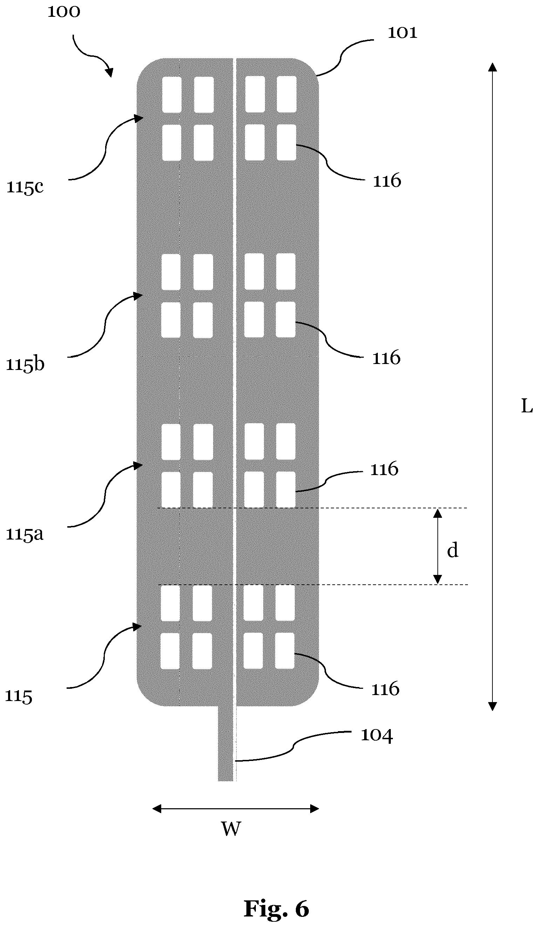

**Fig. 6** shows a schematic illustration of an apparatus 100 for stimulating and / or recording signals traveling along nerve fibers according to yet further aspects of the present disclosure. The apparatus 100 comprises: two or more electrode arrangements 115, 115a, 115b, 115c arranged on an elongated and non-conducting base material 101. In one example, the two or more electrode arrangements could be the first or the second electrode arrangement as described in here. Each electrode arrangement 115, 115a, 115b, 115c depicted in Fig. 6 comprises a plurality of electrodes (one of which is indicated as 116 in each of the electrode arrangements 115, 115a, 115b, 115c). A distance (indicated as "d", i.e., edge to edge as already defined with respect to Fig. 2) between a first electrode arrangement 115 and a neighboring second electrode arrangement 115a of the two or more electrode arrangements is larger than a distance of all neighboring pairs of electrodes of the same electrode arrangement 115, 115a, 115b, 115c.

In one example, the elongated base material 101 comprises an aspect ratio larger than three, preferably larger than four and more preferably larger than five. This means that the length L is larger than three, preferably larger than four and more preferably larger than five times the width W of the elongated base material 101.

The apparatus 100 may comprise at least two, three or four electrode arrangements 115, 115a, 115b, 115c, wherein a distance d between neighboring electrode arrangements 115, 115a in the longitudinal direction of the elongated base material 101 corresponds to a typical distance in a longitudinal direction of the spinal cord between centers of neighboring dorsal root entry zone. Respective advantages are described as set forth elsewhere in here. Further, it is understood that increasing the number of electrode arrangements in the longitudinal direction of the apparatus may lead to an improved stimulation of dermatome zones and, thereby, elicitation of a multitude of perceptual channels in the cortex of the person. This enhances transmission of information. It may be the case that further increasing the number of electrode arrangements 115, 115a, 115b, 115c could make implantation of the apparatus 100 difficult, due to an increased size of the apparatus 100. Thereby, an optimum balance is struck if two, three or four electrode arrangements 115, 115a, 115b, 115c are provided.

As further derivable from Fig. 6, the electrodes 116 of each electrode arrangement 115, 115a, 115b, 115c are arranged substantially as an array having columns and rows. In one example, said array may have two rows and four columns and / or the electrodes 116 in the array may be substantially equidistantly spaced apart, such that each electrode arrangement 115, 115a, 115b, 115c allows to stimulate and record three distinct body dermatomes. As understood, for recording, a reference electrode 110, 111 may be provided as described elsewhere herein in greater detail.

### Tunnel fixation

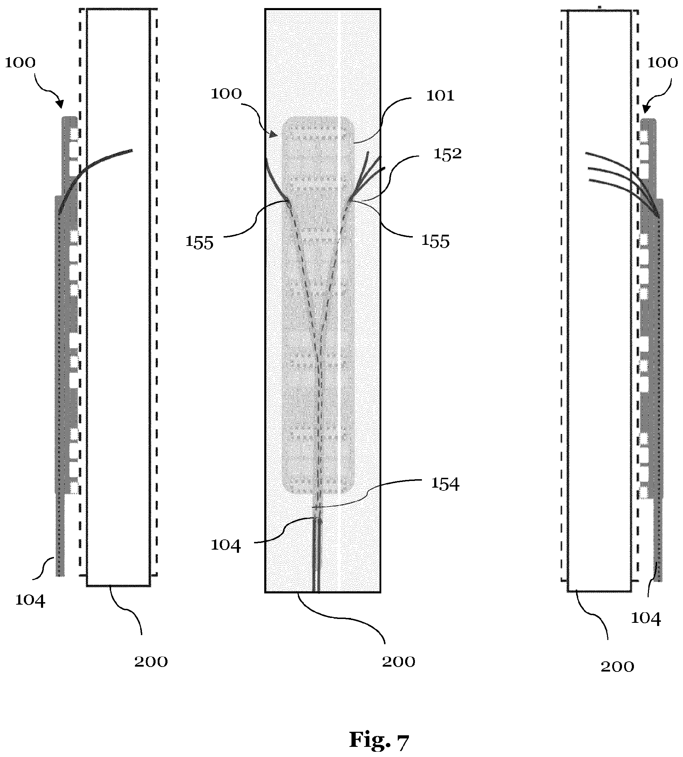

**Fig. 7** shows a schematic illustration of an apparatus 100 for stimulating and / or recording signals traveling along nerve fibers of a spinal cord 200, comprising: one or more electrode arrangements arranged on an elongated and non-conducting base material 101. The elongated base material 101 may comprise a guiding tunnel 154 configured to receive and guide at least one wire element 152, preferably a wire electrode, more preferably to surround the spinal cord at least partially 200 with the at least one wire element 152. Further, a plurality of wire elements 152 may also be received and guided through the guiding tunnel 154. It is understood that the apparatus 100 of Fig. 7 may incorporate the features of the remaining apparatuses described elsewhere herein.

In the left part of Fig. 7, the apparatus 100 and the spinal cord 200 are shown from a right-side view. In the middle part of Fig. 7, the apparatus 100 and the spinal cord 200 are shown from a back view, in which the spinal cord 200 is behind the apparatus 100. This may be understood such that the middle part of Fig. 7 is viewed dorsally at the back side of the apparatus 100. In the right part of Fig. 7, the apparatus 100 and the spinal cord 200 are shown from a left-side view.

The guiding tunnel 154 may form a hollow portion so as to facilitate said receiving and guiding of the at least one wire element 152. The guiding tunnel 154 allows that the at least one wire element 152 can be received so as to improve fixing and maintaining a position of the apparatus 100 during usage.

As can be seen in Fig. 7, the guiding tunnel 154 has at least two exit openings 155 on opposing sides of the width direction of the elongated base material 101. The exit openings 155 are configured to receive and guide at least one wire element 152 (as understood, this may include a plurality of wire elements 152) to form a y-shape such that a first and a second tip of the y-shape can be placed on the distal side of the spinal cord 200. Placing the tip of the at least one wire elements 152 on the distal side of the spinal cord 200 improves fixing of the apparatus 100. Further, it is understood that the y-shape, which is formed by the wire(s) 152 can be disposed partially or fully around the circumference of the spinal cord 200. In one example, the at least one wire element 152 can comprise electrodes, in particular multiple electrodes. For instance, multiple electrodes could be disposed along the length of the at least one wire element 152, exposed outside of the exit openings 155. This may be advantageous, if the at least one wire element 152 is used for stimulation and recording of nerve fibers.

The guiding tunnel 154 may comprise an entry opening, arranged on the ventral side of the base material or on the dorsal side of the base material. The ventral side may typically be the side on which the one or more electrode arrangements are arranged. The dorsal side may be the opposite side of the apparatus 100, e.g. facing the back of the person.

The wires 152 may travel alone by themselves or using some helping means, such as a stylet via the guiding tunnel 154. Preferably, the guiding tunnel 154 is formed using substantially the same or similar insulating material as the one covering the apparatus 100 on the back side, i.e., opposite to the side on which the electrodes are disposed. The exit openings 155 of the guiding tunnel 154 and the angle of the guiding tunnel 154 with respect to the lateral edge of the apparatus 100 is designed so that the wires exit substantially bilaterally to the sides. Alternatively, they may exit longitudinally along the apparatus 100, however, the former configuration of angles is preferred. The wires 152 may be provided with a material having such a stiffness that the wires 152 substantially bend around the spinal cord 200 when the wires 152 substantially enter the epidural space cavity.

One further aspect relates to a system for stimulating and / or recording signals traveling along nerve fibers of a spinal cord, the system comprising the afore described apparatus 100; and at least one partially insulated wire electrode 152 comprising one or more electric contacts configured such that at least some of the electric contacts reside at the left side and / or on the right side and / or on the ventral side of the spinal cord 200, when the wire electrode 152 is inserted into the guiding tunnel 154. Preferably, the at least one partially insulated wire electrode 152 is configured to surround the spinal cord 200 at least partially, such that movement of the apparatus 100 relative to the spinal cord 200 is reduced during ordinary use. Further, the at least one partially insulated wire electrode 152 is configured to provide a restoring force pulling the apparatus 100 towards the spinal cord 200 during ordinary use.

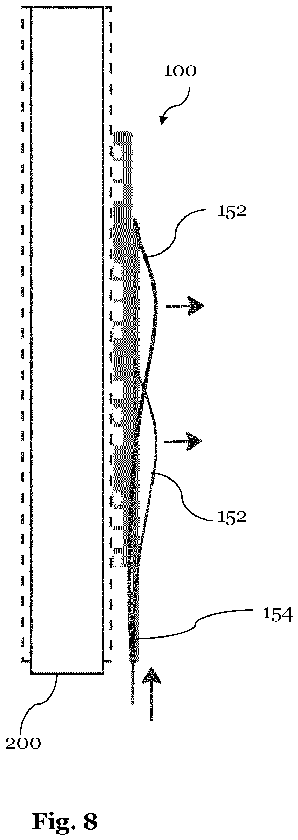

**Fig. 8** shows an apparatus 100 in one example, in which the guiding tunnel 154 comprises one or more elongated openings, arranged on the dorsal side of the base material 101, configured to allow bulging of the one or more wire elements 152 substantially in the dorsal direction (indicated by the arrows to the right side in Fig. 8), when pushed into the guiding tunnel 154 so as to provide a force pushing the apparatus 100 in the ventral direction during use. In this manner, the one or more wire elements 152 may act as spring-loaded wires 152 that coming out at the back side of the base material 101. This stabilizes and / or reduces the distance of the apparatus 100 to the spinal cord 200 by pushing the apparatus 100 ventrally (i.e., in the left direction of Fig. 8). This improves stimulation and recording of nerve fibers. If pushing is not performed in such a manner, it may be the case that a variation in the stimulus intensity and the detected ECAP amplitude is more substantial. Typically, the bulging occurs after the tail of the one or more wires 152 is pushed into the guiding tunnel 154. In one example, such a pushing may be performed by a surgeon. The tip of the one or more wires 152 may be fixed to the base material so as to promote said bulging and the development of spring-loaded wires 152. In a similar fashion, provision of lateral stabilization may be ensured (not expressly shown in Fig. 8). These wires 152 could be on an outer, e.g., an outermost layer of the base material 101. The wires may also provide for stimulation and / or recording. However, it is preferred that these wires primarily serve the purpose of fixation / stabilization. In one example, a section of the wires 152 can also be provided with a material configured to prevent damaging surrounding tissue. For this, the material could be the same or a similar material as the base material 101.

It is understood that the guiding tunnel 154 described in here may expand over at least 20%, preferably at least 30%, preferably at least 40%, preferably at least 50%, preferably at least 60%, preferably at least 70%, preferably at least 80%, preferably at least 90% or more of the length of the base material 101 in the longitudinal direction. The length of the guiding tunnel 154 can depend on the desired configuration for placing the apparatus 100 with respect to the spinal cord 200 to provide for a minimum amount of migration of the apparatus 100 (e.g. to prevent movement of the apparatus 100).

### Wings fixation

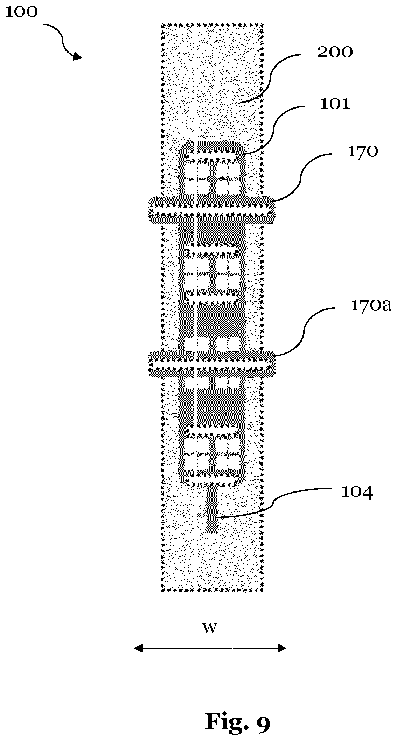

**Fig. 9** shows a schematic illustration of an apparatus 100 for stimulating and / or recording signals traveling along nerve fibers of a spinal cord 200. The apparatus 100 comprises one or more electrode arrangements (as described herein) arranged on an elongated and non-conducting base material 101, and one or more protrusions 170, 170a extending from the base material 101 in a width direction w of the base material 101. The protrusions 170, 170a could have the shape of wings. The one or more protrusions 170, 170a are configured to surround the spinal cord 200 at least partially 200 and comprise one or more electrodes configured to reside at the left side and / or on the right side of the spinal cord 200. Alternatively or additionally, the apparatus 100 comprises one or more protrusions 170, 170a, extending from the base material 101 such that a force is provided pushing the apparatus 100 towards the spinal cord 200 during use. In this manner, the fixation of the apparatus 100 is improved. The wings 170, 170a (these could be regarded as bumps in one example) may bulge out ventrally from the back of the apparatus 100 to act as springs pushing against the spinal cavity wall (i.e., into the image plane of Fig. 9). Thereby, they constantly push the apparatus 100 to the spinal cord 200 in order to stabilize the apparatus 100 in place.

It is understood that the apparatus 100 of Fig. 9 may incorporate the features of the remaining apparatuses described elsewhere herein.

### Surrounding reference electrode

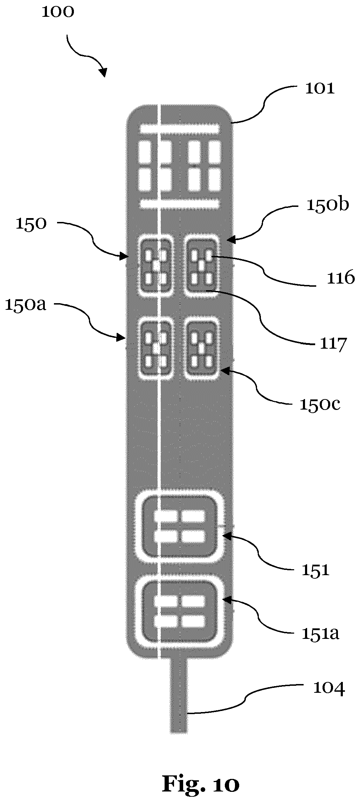

**Fig. 10** shows a schematic illustration of an apparatus 100 for stimulating and / or recording signals traveling along nerve fibers of a spinal cord 200. The apparatus 100 comprises one or more electrode arrangements 150, 150a, 150b, 150c, arranged on an elongated and non-conducting base material 101, each electrode arrangement 150, 150a, 150b, 150c is comprising: a plurality of electrodes (one of which is indicated as 116 in the electrode arrangement 150b); a reference electrode 117 substantially surrounding the plurality of electrodes 116; wherein the reference electrode 117 is configured to provide a reference counter potential for stimulating and / or a reference potential for recording of the nerve fibers of the spinal cord 200 via the plurality of electrodes 116.

It is understood that the apparatus of Fig. 10 may incorporate the features of the remaining apparatuses described elsewhere herein.

It is noted that the dimensions of the electrode arrangements and their plurality of electrodes and the reference electrode in Fig. 10 are of schematic nature. Further, also their relative positioning with respect to one another are of schematic nature. Thereby, the arrangement in Fig. 10 is not to be understood limiting in any sense. Rather, any kind of dimensions and / or relative positionings with respect to one another may be encompassed. For instance, the apparatus 100 may comprise larger or smaller separations between electrode arrangements. The selection of the dimensions and / or their relations may typically be performed so as to increase a resolution for stimulation and / or recording. Further, as shown in a bottom portion of the apparatus 100 in Fig. 10, two additional electrode arrangements 151, 151a are exemplarily depicted. Also their dimensions and / or relative positioning with respect to other electrode arrangements of the apparatus 100 can be varied. For instance, the distance between electrode arrangement 151 and / or 151a and one or more of electrode arrangements 150, 150a, 150b, and 150c along the apparatus 100 could be smaller or even larger than depicted. In one example, it is beneficial to provide a certain distance between electrode arrangement 151 and / or 151a and one or more of electrode arrangements 150, 150a, 150b, and 150c so as to improve detection of ECAPs (by having a certain distance to electrode arrangements for stimulation).

In this manner, multiple dermatome zones may be stimulated and / or recorded in a more-isolated manner. It is possible to switch between different ones of the one or more electrode arrangements 150, 150a, 150b, 150c such that an optimum stimulation and / or recording configuration may be reached. This significantly increases the stimulation capabilities and encoding of information for the person. The apparatus 100 of Fig. 10 enables capturing ECAPs travelling in various propagation directions in the spinal cord. This is attributable to the reference electrode 117, which is surrounding the recording / stimulation electrodes 116 in substantially all directions. Conventional designs do not reveal such features, and ECAPs may be at best only detected in case the recording reference and recording electrode are located along a voltage gradient of the ECAP. For instance, if ECAPs are travelling cranially, the equipotential medio-lateral contact pairs cannot detect any voltage difference in conventional designs.

In the afore-mentioned embodiments and aspects, it is possible that some electrode arrangements arranged on caudal, rostral, medial and / or lateral portions of the base material 101 of the apparatus 100 serve for stimulation and / or ECAP detection while other electrode arrangements serve for therapeutic stimulation. In one example, electrode arrangements in one portion (e.g. a caudal end) are used for stimulation and electrode arrangements in another portion (e.g. a rostral end) are used for ECAP detection. Such a configuration may serve as range-finder in a way that if a detected ECAP magnitude is reduced in a specific column of electrodes of the apparatus 100 (e.g. medial or lateral columns) then as a response, stimulation can be adjusted in such a way to increase the magnitude of ECAP and restore it to initial value.

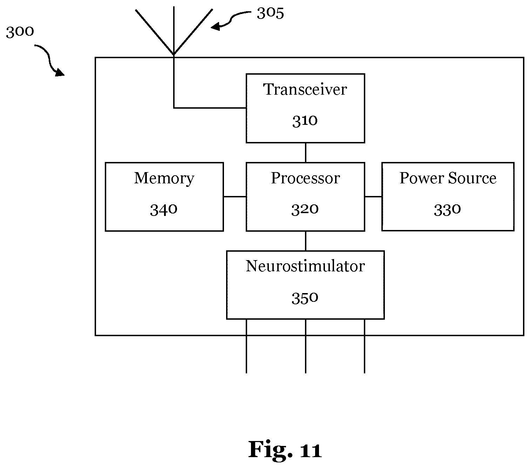

**Fig. 11** shows a functional block circuit diagram illustrating a neurostimulation device 300 according to aspects of the present disclosure. The neurostimulation device 300 may comprise a communication antenna 305 operably connected to a transceiver 310, configured for wireless communication (e.g. via NFC, Bluetooth or a similar wireless communication technology). Additionally or alternatively, one or more wires may be in communication with the transceiver 310.

The transceiver 310 may be configured to receive neurostimulation signals and / or recorded ECAPs (e.g. from a recording apparatus as described herein). The transceiver 310 may be operably connected to a processor 320, which may be configured to process or generate the neurostimulation signals, e.g., based on recorded ECAPs and / or on instructions received via the transceiver and / or stored and obtained in a memory 340. The memory 340, such as a non-transitory computer-readable memory may also be configured to store one or more neurostimulation signals, e.g. based on recorded ECAPs. The memory 340 may be operably connected to the processor 320. The processor 320 may also receive neurostimulation signals from the memory 340.

The neurostimulation device 300 may further comprise a neurostimulator 350, that receives the generated neurostimulation signals from the processor 320. The neurostimulator 350 may also be connected to one or more output signal leads 360 that may be in conductive communication with the apparatus 100 as described herein for applying the neurostimulation signals to the CNS of a person. In one example, the processor 320 may not be comprised by the neurostimulation device 300 but arranged at a different location. In such an example, a wireless communication between the neurostimulation device 300 and the processor 320 may be configured to process or generate the neurostimulation signals and send the neurostimulation signals to the neurostimulation device 300 via wireless communication.

The neurostimulation device 300 may also comprise a rechargeable power source 330, which may for instance be wirelessly charged via a wireless charging interface. According to some aspects of the present disclosure, the processor 320 may thus be operably connected to neurostimulation circuitry adapted to generate and apply a plurality of neurostimulation signals (or sequences) to a central nervous system of a person.

### Computer program

In aspects relating to the computer program described herein, the instructions for causing the analyzing of the recorded antidromic ECAP comprise instructions for causing comparing the recorded antidromic ECAP to a reference value or signal; and the instructions for outputting the second neurostimulation signal comprises instructions for modifying one or more signal parameters of the first neurostimulation signal to obtain the second neurostimulation signal.

Further, the computer program described herein may comprise instructions for recording, from a third electrode arrangement of the spinal cord stimulation and recording apparatus, an ECAP, propagating orthodromically along the stimulated afferent nerve fibers. Further, analyzing the recorded antidromic ECAP may comprise comparing the recorded antidromic ECAP to the recorded orthodromic ECAP.

Further, in aspects of the computer program described herein, analyzing the recorded antidromic ECAP may comprise determining a dermatome corresponding to a subset or all of the stimulated afferent nerve fibers.

Further, in aspects of the computer program described herein, determining the dermatome corresponding to the subset or all of the stimulated afferent nerve fibers comprises estimating a position of the subset or all of the stimulated afferent nerve fibers within the spinal cord.

Further, in aspects of the computer program described herein, estimating the position of the subset or all of the stimulated afferent nerve fibers within the spinal cord comprises comparing signal amplitudes of two more recording electrodes of the second electrode arrangement.

### 6. List of reference signs

- 400: apparatus
- 101: base material
- 104: lead
- 110: first central reference electrode
- 111: second central reference electrode
- 115, 115a, 115b, 115c: plurality of first electrodes
- 116: electrode
- 117: reference electrode
- 120, 120a: plurality of second electrodes
- 121: electrode
- 130, 130a: one or more first electrode arrangement(s)
- 140, 140a, 140b: one or more second electrode arrangements(s)
- 150, 150a, 150b, 150c: one or more electrode arrangements(s)
- 151, 151a: one or more electrode arrangements(s)
- 152: wire
- 154: tunnel
- 155: tunnel opening
- 160: multiplexer
- 170, 170a: protrusions

- 200: spinal cord

- 300: neurostimulation device
- 305: communication antenna
- 310: transceiver of the neurostimulation device
- 320: processor of the neurostimulation device
- 330: power source of the neurostimulation device
- 340: memory of the neurostimulation device
- 350: neurostimulator of the neurostimulation device

- 360: leads

- 400: person

- 500: method
- 600: system

- 700: programmer device

- d: distance between a center of a first dorsal root entry zone and a second dorsal root entry zone
- w: width/width direction
- W: width of the elongated base material
- L: length of the elongated base material

## Claims

1. Apparatus for stimulating and / or recording signals travelling along nerve fibers of a spinal cord,
comprising one or more first electrode arrangements arranged on an elongated and non-conducting base material, each first electrode arrangement comprising:
an elongated first central reference electrode extending along a longitudinal direction of the elongated base material; and
a plurality of first electrodes arranged on both sides of the elongated first central reference electrode;
wherein a length of the elongated first central reference electrode is at least two times larger or at least three times larger than an average length of the plurality of first electrodes; and
wherein the elongated first central reference electrode is configured to provide a counter potential for stimulating and / or a reference potential for recording of the signals travelling along nerve fibers of the spinal cord via the plurality of first electrodes,
wherein the apparatus is further comprising one or more second electrode arrangements arranged on the elongated base material, each second electrode arrangement comprising:
an elongated second central reference electrode extending along a width direction of the elongated base material, substantially perpendicular to the longitudinal
direction of the elongated base material; and
a plurality of second electrodes arranged in one or more rows on one or on both sides of the elongated second central reference electrode;
wherein a distance between the second electrodes and the elongated second central reference electrode is substantially the same for all second
electrodes of the same row.

2. The apparatus according to claim 1, wherein each electrode of the plurality of first electrodes comprise an elongated shape and wherein the longitudinal direction of each electrode of the plurality of first electrodes is substantially parallel to the longitudinal direction of the first central reference electrode.

3. The apparatus according to claim 1 or 2, wherein the plurality of first electrodes is arranged in a line-symmetric manner with respect to a central line of the elongated first central reference electrode; and / or wherein each first electrode arrangement comprises three or more pairs of first electrodes on both sides of the first central reference electrode.

4. The apparatus according to any of claims 1 to 3, wherein each electrode of the plurality of first electrodes can be controlled independently to stimulate and / or record from the nerve fibers of the spinal cord.

5. The apparatus according to any of the preceding claims 1 to 4, comprising at least two first electrode arrangements, wherein a distance between the two first electrode arrangements in the longitudinal direction of the elongated base material
corresponds to a typical distance, in a longitudinal direction of the spinal cord,
between a center of a first dorsal root entry zone and a second dorsal root entry zone.

6. The apparatus according to any one of claims 1 to 5,
wherein the first electrode arrangement is configured for stimulating afferent sensory nerve fibers of the spinal cord; and/or wherein the second electrode arrangement is configured for recording evoked compound action potentials, ECAPs, elicited via the first electrode arrangement;
wherein, optionally, the second electrode arrangement is configured for stimulating afferent sensory nerve fibers of the spinal cord; and/or
wherein the first electrode arrangement is configured for recording evoked compound action potentials, ECAPs, elicited via the second electrode arrangement.

7. An apparatus for stimulating and / or recording signals travelling along nerve fibers of a spinal cord,
comprising one or more second electrode arrangements arranged on an elongated and non-conducting base material, each second electrode arrangement comprising:
an elongated second central reference electrode extending along a width direction of the elongated base material, substantially perpendicular to a longitudinal direction of the elongated base material; and
a plurality of second electrodes arranged in one or more rows on one or on both sides of the second elongated central reference electrode;
wherein a distance between the second electrodes and the elongated second central reference electrode is substantially the same for all second electrodes of the same row,
wherein each electrode of the plurality of second electrodes comprise an elongated shape and wherein the longitudinal direction of each electrode of the plurality of second electrodes is substantially orthogonal to the longitudinal direction of the second central reference electrode,
optionally, wherein the plurality of second electrodes is arranged in a line-symmetric manner with respect to a central line of the elongated second central reference electrode; and / or wherein each row of the second electrode arrangement comprises at least three, or at least five second electrodes being arranged across a central line of the spinal cord.

8. The apparatus according to claim 7, wherein each second electrode can be controlled independently for stimulating and / or recording of the nerve fibers of the spinal cord.

9. The apparatus according to claim 7 or 8, comprising at
least two second electrode arrangements,
wherein a distance between the two second electrode arrangements in the longitudinal direction of the elongated base material corresponds to a typical distance, in a longitudinal direction of the spinal cord, between a center of a first dorsal root entry zone and a second dorsal root entry zone.

10. The apparatus according to any of the preceding claims 7 to 9,
further comprising one or more first electrode arrangements arranged on the elongated base material, each first electrode arrangement comprising:
an elongated first central reference electrode extending along a longitudinal direction of the elongated base material; and
a plurality of first electrodes arranged on both sides of the elongated first central reference electrode;
wherein a length of the elongated first central reference electrode is at least two times larger or at least three times larger than an average length of the plurality of first electrodes; and
wherein the elongated first central reference electrode is configured to provide a reference potential for stimulating and / or for recording of the signals travelling along nerve fibers of the spinal cord via the plurality of first electrodes.

11. A neurostimulation device for stimulating and/or recording signals travelling along nerve fibers of a spinal cord, the device comprising:
the apparatus of any of the preceding claims; and
neurostimulation and recording circuitry operably connected to the electrodes of the apparatus.

12. A computer program, comprising instructions for causing a neurostimulation device according to claim 11 to carry out the following steps when being executed by signal processing circuitry of the neurostimulation device:
outputting a first neurostimulation signal to a first electrode arrangement of a spinal cord stimulation and recording apparatus, wherein the neurostimulation signal is configured to elicit action potentials in afferent sensory nerve fibers of a spinal cord;
recording, from a second electrode arrangement of the spinal cord stimulation and recording apparatus, an evoked compound action potential, ECAP, propagating antidromically along the stimulated afferent nerve fibers;
analyzing the recorded antidromic ECAP; and
outputting, based on the analyzed and recorded antidromic ECAP, a second neurostimulation signal to the first electrode arrangement, wherein the second neurostimulation signal is configured to elicit action potentials in the afferent sensory nerve fibers of the spinal cord.

13. Computer program of claim 12,
wherein the instructions for causing the analyzing of the recorded antidromic ECAP comprise instructions for causing comparing the recorded antidromic ECAP to a reference value or signal; and
wherein the instructions for outputting the second neurostimulation signal comprises instructions for modifying one or more signal parameters of the first neurostimulation signal to obtain the second neurostimulation signal.

## Patentansprüche

1. Vorrichtung zum Stimulieren und/oder Aufzeichnen von Signalen, die sich entlang von Nervenfasern eines Rückenmarks bewegen, umfassend eine oder mehrere erste Elektrodenanordnungen, die auf einem länglichen und nichtleitenden Basismaterial angeordnet sind, wobei jede erste Elektrodenanordnung umfasst:
eine längliche erste zentrale Referenzelektrode, die sich entlang einer Längsrichtung des länglichen Basismaterials erstreckt; und
eine Vielzahl von ersten Elektroden, die auf beiden Seiten der länglichen ersten zentralen Referenzelektrode angeordnet sind;
wobei eine Länge der länglichen ersten zentralen Referenzelektrode mindestens zweimal größer oder mindestens dreimal größer ist als eine durchschnittliche Länge der Vielzahl von ersten Elektroden; und
wobei die längliche erste zentrale Referenzelektrode dazu konfiguriert ist, ein Gegenpotential zum Stimulieren und/oder ein Referenzpotential zum Aufzeichnen der Signale, die sich entlang von Nervenfasern des Rückenmarks bewegen, über die Vielzahl von ersten Elektroden bereitzustellen,
wobei die Vorrichtung ferner eine oder mehrere zweite Elektrodenanordnungen umfasst, die auf dem länglichen Basismaterial angeordnet sind, wobei jede zweite Elektrodenanordnung umfasst:
eine längliche zweite zentrale Referenzelektrode, die sich entlang einer Breitenrichtung des länglichen Basismaterials, substantiell senkrecht zur Längsrichtung des länglichen Basismaterials, erstreckt; und
eine Vielzahl von zweiten Elektroden, die in einer oder mehreren Reihen auf einer oder auf beiden Seiten der länglichen zweiten zentralen Referenzelektrode angeordnet sind;
wobei ein Abstand zwischen den zweiten Elektroden und der länglichen zweiten zentralen Referenzelektrode für alle zweiten Elektroden derselben Reihe substantiell gleich ist.

2. Vorrichtung nach Anspruch 1, wobei jede Elektrode der Vielzahl von ersten Elektroden eine längliche Form aufweist und wobei die Längsrichtung jeder Elektrode der Vielzahl von ersten Elektroden substantiell parallel zur Längsrichtung der ersten zentralen Referenzelektrode ist.

3. Vorrichtung nach Anspruch 1 oder 2, wobei die Vielzahl von ersten Elektroden in einer liniensymmetrischen Weise in Bezug auf eine Mittellinie der länglichen ersten zentralen Referenzelektrode angeordnet ist; und/oder wobei jede erste Elektrodenanordnung drei oder mehr Paare von ersten Elektroden auf beiden Seiten der ersten zentralen Referenzelektrode umfasst.

4. Vorrichtung nach einem der Ansprüche 1 bis 3, wobei jede Elektrode der Vielzahl von ersten Elektroden unabhängig gesteuert werden kann, um die Nervenfasern des Rückenmarks zu stimulieren und/oder von ihnen aufzuzeichnen.

5. Vorrichtung nach einem der vorhergehenden Ansprüche 1 bis 4, umfassend mindestens zwei erste Elektrodenanordnungen, wobei ein Abstand zwischen den beiden ersten Elektrodenanordnungen in der Längsrichtung des länglichen Basismaterials einem typischen Abstand, in einer Längsrichtung des Rückenmarks, zwischen einem Zentrum einer ersten Hinterwurzeleintrittszone und einer zweiten Hinterwurzeleintrittszone entspricht.

6. Vorrichtung nach einem der Ansprüche 1 bis 5,
wobei die erste Elektrodenanordnung zum Stimulieren von afferenten sensorischen Nervenfasern des Rückenmarks konfiguriert ist; und/oder wobei die zweite Elektrodenanordnung zum Aufzeichnen von evozierten Summenaktionspotentialen, ECAPs, die über die erste Elektrodenanordnung hervorgerufen werden, konfiguriert ist;
wobei, optional, die zweite Elektrodenanordnung zum Stimulieren von afferenten sensorischen Nervenfasern des Rückenmarks konfiguriert ist; und/oder
wobei die erste Elektrodenanordnung zum Aufzeichnen von evozierten Summenaktionspotentialen, ECAPs, die über die zweite Elektrodenanordnung hervorgerufen werden, konfiguriert ist.

7. Vorrichtung zum Stimulieren und/oder Aufzeichnen von Signalen, die sich entlang von Nervenfasern eines Rückenmarks bewegen,
umfassend eine oder mehrere zweite Elektrodenanordnungen, die auf einem länglichen und nichtleitenden Basismaterial angeordnet sind,
wobei jede zweite Elektrodenanordnung umfasst:
eine längliche zweite zentrale Referenzelektrode, die sich entlang einer Breitenrichtung des länglichen Basismaterials, substantiell senkrecht zu einer Längsrichtung des länglichen Basismaterials, erstreckt; und
eine Vielzahl von zweiten Elektroden, die in einer oder mehreren Reihen auf einer oder auf beiden Seiten der zweiten länglichen zentralen Referenzelektrode angeordnet sind;
wobei ein Abstand zwischen den zweiten Elektroden und der länglichen zweiten zentralen Referenzelektrode für alle zweiten Elektroden derselben Reihe substantiell gleich ist,
wobei jede Elektrode der Vielzahl von zweiten Elektroden eine längliche Form aufweist und wobei die Längsrichtung jeder Elektrode der Vielzahl von zweiten Elektroden substantiell orthogonal zur Längsrichtung der zweiten zentralen Referenzelektrode ist,
optional, wobei die Vielzahl von zweiten Elektroden in einer liniensymmetrischen Weise in Bezug auf eine Mittellinie der länglichen zweiten zentralen Referenzelektrode angeordnet ist; und/oder wobei jede Reihe der zweiten Elektrodenanordnung mindestens drei oder mindestens fünf zweite Elektroden umfasst, die über eine Mittellinie des Rückenmarks hinweg angeordnet sind.

8. Vorrichtung nach Anspruch 7, wobei jede zweite Elektrode unabhängig zum Stimulieren und/oder Aufzeichnen der Nervenfasern des Rückenmarks gesteuert werden kann.

9. Vorrichtung nach Anspruch 7 oder 8, umfassend mindestens zwei zweite Elektrodenanordnungen,
wobei ein Abstand zwischen den beiden zweiten Elektrodenanordnungen in der Längsrichtung des länglichen Basismaterials einem typischen Abstand, in einer Längsrichtung des Rückenmarks, zwischen einem Zentrum einer ersten Hinterwurzeleintrittszone und einer zweiten Hinterwurzeleintrittszone entspricht.

10. Vorrichtung nach einem der vorhergehenden Ansprüche 7 bis 9,
ferner umfassend eine oder mehrere erste Elektrodenanordnungen, die auf dem länglichen Basismaterial angeordnet sind, wobei jede erste Elektrodenanordnung umfasst:
eine längliche erste zentrale Referenzelektrode, die sich entlang einer Längsrichtung des länglichen Basismaterials erstreckt; und
eine Vielzahl von ersten Elektroden, die auf beiden Seiten der länglichen ersten zentralen Referenzelektrode angeordnet sind;
wobei eine Länge der länglichen ersten zentralen Referenzelektrode mindestens zweimal größer oder mindestens dreimal größer ist als eine durchschnittliche Länge der Vielzahl von ersten Elektroden; und
wobei die längliche erste zentrale Referenzelektrode dazu konfiguriert ist, ein Referenzpotential zum Stimulieren und/oder zum Aufzeichnen der Signale, die sich entlang von Nervenfasern des Rückenmarks bewegen, über die Vielzahl von ersten Elektroden bereitzustellen.

11. Neurostimulationsvorrichtung zum Stimulieren und/oder Aufzeichnen von Signalen, die sich entlang von Nervenfasern eines Rückenmarks bewegen, wobei die Vorrichtung umfasst:
die Vorrichtung nach einem der vorhergehenden Ansprüche; und
eine Neurostimulations- und Aufzeichnungsschaltung, die operabel mit den Elektroden der Vorrichtung verbunden ist.

12. Computerprogramm, umfassend Anweisungen, um eine Neurostimulationsvorrichtung nach Anspruch 11 zu veranlassen, die folgenden Schritte auszuführen, wenn sie von einer Signalverarbeitungsschaltung der Neurostimulationsvorrichtung ausgeführt werden:
Ausgeben eines ersten Neurostimulationssignals an eine erste Elektrodenanordnung einer Rückenmarkstimulations- und - aufzeichnungsvorrichtung, wobei das Neurostimulationssignal dazu konfiguriert ist, Aktionspotentiale in afferenten sensorischen Nervenfasern eines Rückenmarks hervorzurufen;
Aufzeichnen, von einer zweiten Elektrodenanordnung der Rückenmarkstimulations- und -aufzeichnungsvorrichtung, eines evozierten Summenaktionspotentials, ECAP, das sich antidromisch entlang der stimulierten afferenten Nervenfasern ausbreitet;
Analysieren des aufgezeichneten antidromischen ECAP; und
Ausgeben, basierend auf dem analysierten und aufgezeichneten antidromischen ECAP, eines zweiten Neurostimulationssignals an die erste Elektrodenanordnung, wobei das zweite Neurostimulationssignal dazu konfiguriert ist, Aktionspotentiale in den afferenten sensorischen Nervenfasern des Rückenmarks hervorzurufen.

13. Computerprogramm nach Anspruch 12,
wobei die Anweisungen zum Veranlassen des Analysierens des aufgezeichneten antidromischen ECAP Anweisungen zum Veranlassen des Vergleichens des aufgezeichneten antidromischen ECAP mit einem Referenzwert oder -signal umfassen; und
wobei die Anweisungen zum Ausgeben des zweiten Neurostimulationssignals Anweisungen zum Modifizieren eines oder mehrerer Signalparameter des ersten Neurostimulationssignals umfassen, um das zweite Neurostimulationssignal zu erhalten.

## Revendications

1. Appareil pour la stimulation et/ou l'enregistrement de signaux se propageant le long de fibres nerveuses d'une moelle épinière,
comprenant un ou plusieurs agencements d'électrodes agencées sur un matériau de base allongé et non conducteur, chaque premier agencement d'électrodes comprenant :
une première électrode de référence centrale allongée s'étendant le long d'une direction longitudinale du matériau de base allongé ; et
une pluralité de premières électrodes agencées des deux côtés de la première électrode de référence centrale allongée ;
dans lequel une longueur de la première électrode de référence centrale allongée est au moins deux fois supérieure ou au moins trois fois supérieure à une longueur moyenne de la pluralité de premières électrodes ; et
dans lequel la première électrode de référence centrale allongée est configurée pour produire un contre-potentiel pour la stimulation, et/ou un potentiel de référence pour l'enregistrement des signaux se propageant le long de fibres nerveuses de la moelle épinière via la pluralité de premières électrodes,
dans lequel l'appareil comprend en outre une ou plusieurs seconds agencements d'électrodes agencées sur le matériau de base allongé, chaque second agencement d'électrodes comprenant :
une seconde électrode de référence centrale allongée s'étendant le long d'une direction de la largeur du matériau de base allongé, sensiblement perpendiculaire à la direction longitudinale du matériau de base allongé ; et
une pluralité de secondes électrodes agencées en une ou plusieurs rangées sur l'un ou sur les deux côtés de la seconde électrode de référence centrale allongée ;
dans lequel une distance entre les secondes électrodes et la seconde électrode de référence centrale allongée est sensiblement la même pour toutes les secondes électrodes de la même rangée.

2. L'appareil selon la revendication 1, dans lequel chaque électrode de la pluralité de premières électrodes présente une forme allongée, et dans lequel la direction longitudinale de chaque électrode de la pluralité de premières électrodes est sensiblement parallèle à la direction longitudinale de la première électrode centrale de référence.

3. L'appareil selon la revendication 1 ou 2, dans lequel la pluralité de premières électrodes sont agencées avec symétrie axiale par rapport à un axe central de la première électrode de référence centrale allongée ; et/ou dans lequel chaque premier agencement d'électrode comprend trois ou plus paires de premières électrodes des deux côtés de la première électrode centrale de référence.

4. L'appareil selon l'une des revendications 1 à 3, dans lequel chaque électrode de la pluralité de premières électrodes peut être indépendamment contrôlée pour stimuler et/ou enregistrer à partir des fibres nerveuses de la moelle épinière.

5. L'appareil selon l'une des revendications 1 à 4 précédentes, comprenant au moins deux premiers agencements d'électrodes, dans lequel une distance entre les deux premiers agencements d'électrodes dans la direction longitudinale du matériau de base correspond à une distance typique, dans une direction longitudinale de la moelle épinière, entre un centre d'une première zone d'accès à une racine dorsale et une seconde zone d'accès à une racine dorsale.

6. L'appareil selon l'une des revendications 1 à 5,
dans lequel le premier agencement d'électrodes est configuré pour stimuler des fibres nerveuses sensitives afférentes de la moelle épinière ; et/ou dans lequel le second agencement d'électrodes est configuré pour enregistrer des potentiels d'action composite évoqués, ECAP, induits via le premier agencement d'électrodes ;
dans lequel, éventuellement, le second agencement d'électrodes est configuré pour stimuler des fibres nerveuses sensitives afférentes de la moelle épinière ; et/ou
dans lequel le premier agencement d'électrodes est configuré pour enregistrer des potentiels d'action composite évoqués, ECAP, induits via le second agencement d'électrodes.

7. Un appareil pour la stimulation et/ou l'enregistrement de signaux se propageant le long de fibres nerveuses d'une moelle épinière,
comprenant un ou plusieurs seconds agencements d'électrodes agencées sur un matériau de base allongé et non conducteur, chaque second agencement d'électrodes comprenant :
une seconde électrode de référence centrale allongée s'étendant le long d'une direction de la largeur du matériau de base allongé, sensiblement perpendiculaire à une direction longitudinale du matériau de base allongé ; et
une pluralité de secondes électrodes agencées en une ou plusieurs rangées sur l'un ou sur les deux côtés de la seconde électrode de référence centrale allongée ;
dans lequel une distance entre les secondes électrodes et la seconde électrode de référence centrale allongée est sensiblement la même pour toutes les secondes électrodes de la même rangée,
dans lequel chaque électrode de la pluralité des secondes électrodes présente une forme allongée et dans lequel la direction longitudinale de chaque électrode de la pluralité de secondes électrodes est sensiblement perpendiculaire à la direction longitudinale de la seconde électrode centrale de référence,
éventuellement dans lequel la pluralité de secondes électrodes sont agencées en symétrie axiale par rapport à un axe central de la seconde électrode de référence centrale allongée ; et/ou dans lequel chaque rangée du second agencement d'électrodes comprend au moins trois, ou au moins cinq, secondes électrodes qui sont agencées en travers d'un axe central de la moelle épinière.

8. L'appareil selon la revendication 7, dans lequel chaque seconde électrode peut être indépendamment contrôlée pour la stimulation et/ou l'enregistrement des fibres nerveuses de la moelle épinière.

9. L'appareil selon la revendication 7 ou 8, comprenant au moins deux seconds agencements d'électrodes,
dans lequel une distance entre les deux seconds agencements d'électrodes dans la direction longitudinale du matériau de base allongé correspond à une distance typique,
dans une direction longitudinale de la moelle épinière, entre un centre d'une première zone d'accès à une racine dorsale et une seconde zone d'accès à une racine dorsale.

10. L'appareil selon l'une des revendications 7 à 9 précédentes, comprenant en outre un ou plusieurs premiers agencements d'électrodes agencées sur le matériau de base allongé, chaque premier agencement d'électrodes comprenant :
une première électrode centrale de référence allongée s'étendant le long d'une direction longitudinale du matériau de base allongé ; et
une pluralité de premières électrodes agencées des deux côtés de la première électrode de référence centrale allongée ;
dans lequel une longueur de la première électrode de référence centrale allongée est au moins deux fois supérieure ou au moins trois fois supérieure à une longueur moyenne de la pluralité de premières électrodes ; et
dans lequel la première électrode de référence centrale allongée est configurée pour procurer un potentiel de référence pour la stimulation et/ou pour l'enregistrement des signaux se propageant le long de fibres nerveuses de la moelle épinière via la pluralité des premières électrodes.

11. Un dispositif de neurostimulation pour la stimulation et/ou l'enregistrement de signaux se propageant le long de fibres nerveuses d'une moelle épinière, le dispositif comprenant :
l'appareil de l'une des revendications précédentes ; et
une circuiterie de neurostimulation et d'enregistrement fonctionnellement connectée aux électrodes de l'appareil.

12. Un programme informatique comprenant des instructions pour faire en sorte, lorsqu'elles sont exécutées par une circuiterie de traitement du signal du dispositif de neurostimulation, qu'un dispositif de neurostimulation selon la revendication 11 effectue les étapes suivantes :
la délivrance d'un premier signal de neurostimulation à un premier agencement d'électrodes d'un appareil d'enregistrement et de stimulation de la moelle épinière, le signal de neurostimulation étant configuré pour induire des potentiels d'action dans des fibres nerveuses sensorielles afférentes d'une moelle épinière ;
l'enregistrement, à partir d'un second agencement d'électrodes de l'appareil d'enregistrement et de stimulation de la moelle épinière, d'un potentiel d'action composite évoqué, ECAP, se propageant de façon antidromique le long des fibres nerveuses afférentes stimulées ;
l'analyse de l'ECAP antidromique enregistré ; et
la délivrance au premier agencement d'électrodes, sur la base de l'ECAP antidromique enregistré et analysé, d'un second signal de neurostimulation, le second signal de neurostimulation étant configuré pour induire des potentiels d'action dans les fibres nerveuses sensorielles afférentes de la moelle épinière.

13. Le programme informatique de la revendication 12,
dans lequel les instructions pour faire en sorte d'analyser l'ECAP antidromique enregistré comprennent des instructions pour faire en sorte de comparer l'ECAP antidromique enregistré à un signal ou valeur de référence ; et
dans lequel les instructions pour la délivrance du second signal de neurostimulation comprennent des instructions pour modifier un ou plusieurs paramètres de signal du premier signal de neurostimulation pour obtenir le second signal de neurostimulation.
